# FASCICULE DE BREVET EUROPEEN

(11) **EP 1 993 587 B1**
(45) Date de publication et mention de la délivrance du brevet: **19.10.2011**
(21) Numéro de dépôt: 07731062.1
(22) Date de dépôt: 28.02.2007
(51) Int. Cl.: A61K 38/17, A61P 25/22

(54) **UTILISATION DE LA PROTEINE A HOMEODOMAINE ENGRAILED COMME ANXIOLYTIQUE**
VERWENDUNG DES ENGRAILED HOMEODOMÄNEN-PROTEINS ALS ANXIOLYTIKUM
USE OF THE ENGRAILED HOMEODOMAIN PROTEIN AS AN ANXIOLYTIC

(30) Priorité: 28.02.2006 FR 0601749
(43) Date de publication de la demande: 26.11.2008
(73) Titulaire: Centre National de la Recherche Scientifique, 75016 Paris (FR)
(72) Inventeur: PROCHIANTZ, Alain, F-75006 Paris (FR); VOLOVITCH, Michel, F-75005 Paris (FR)
(74) Mandataire: Vialle-Presles, Marie José
(86) Numéro de dépôt international: PCT/FR2007/000360
(87) Numéro de publication internationale: WO 2007/099227

(56) Documents cités:
- WO-A-2005/007812
- FR-A- 2 662 698
- FR-A- 2 855 178
- SONNIER LAURE ET AL: "Progressive loss of dopaminergic neurons in the ventral midbrain of adult mice heterozygote for Engrailed1." THE JOURNAL OF NEUROSCIENCE : THE OFFICIAL JOURNAL OF THE SOCIETY FOR NEUROSCIENCE 31 JAN 2007, vol. 27, no. 5, 31 janvier 2007 (2007-01-31), pages 1063-1071, XP002445228 ISSN: 1529-2401

## Description

La présente invention concerne l'utilisation de facteurs de transcription Engrailed pour augmenter le métabolisme dopaminergique, et notamment comme anxiolytiques.

Les protéines Engrailed sont des facteurs de transcription de la classe des protéines à homéodomaine. Les mammifères possèdent deux gènes Engrailed : Engrailed-1 et Engrailed-2 ; -les deux protéines correspondantes qui ont une activité biologique similaire, seront collectivement désignées ci-après sous le terme général d'Engrailed (EN).

Chez le nouveau né et chez l'adulte, EN est exprimé dans les neurones dopaminergiques (DA) de la substance noire (qui dégénèrent dans la maladie de Parkinson), dans les cellules des grains du cervelet et dans les noyaux dopaminergiques mésencéphaliques qui jouent un rôle important dans la régulation de l'humeur et la mise en place des comportements addictifs.

Il a été montré (SIMON et al. J Neurosci. 21 (9) : 3126-34, 2001) que la perte de EN au cours du développement est suivie assez rapidement par la dégénérescence des neurones dopaminergiques, et que l'une des cibles transcriptionnelles de EN est l'alpha-synucléine, dont la liaison génétique avec certaines formes familiales de la maladie de Parkinsons a été montrée (POLYMEROPOULOS et al., Science. 276, 2045-7, 1997), et qui constituerait un régulateur négatif de la transmission dopaminergique (ABELIOVICH et al., Neuron, 25, 239-52, 2000).

L'ensemble de ces observations suggère fortement l'implication d'EN dans des pathologies neurodégénératives, et notamment dans des pathologies affectant les neurones dopaminergiques, telles que la maladie de Parkinson. Il a été proposé de réguler l'activité d'EN pour prévenir ces pathologies ou ralentir leur évolution. Ainsi, la Demande PCT WO 2004/104030 décrit des peptides modulateurs de l'activité d'Engrailed, et propose leur utilisation pour prévenir ou traiter des pathologies neurodégénératives.

Les Inventeurs ont maintenant découvert qu'outre son activité sur la survie neuronale, EN possédait également une action directe sur le métabolisme dopaminergique.

Ils ont observé que l'administration d'Engrailed à des souris induisait chez celles-ci un effet anxiolytique, qui s'accompagnait d'une augmentation importante du rapport DOPAC/DA, ce qui traduit un accroissement du métabolisme de la dopamine, dans le striatum, ainsi que d'une hausse de la sérotonine corticale.

La présente invention a en conséquence pour objet l'utilisation d'une protéine Engrailed pour l'obtention d'un médicament anxiolytique.

Comme indiqué ci-dessus, on désigne ici par « protéine Engrailed » une quelconque des protéines Engrailed-1 ou Engrailed-2 d'un vertébré. De préférence, on choisira une protéine Engrailed de l'espèce à laquelle appartient le sujet à traiter.

Avantageusement, ledit médicament est une préparation injectable, de préférence par voie intra-veineuse.

La présente invention sera mieux comprise à l'aide du complément de description qui va suivre, qui se réfère à des exemples illustrant les propriétés anxiolytiques d'Engrailed, et son effet sur le métabolisme dopaminergique.

### EXEMPLE 1 :

Les effets d'une injection journalière d'EN2 à la dose de 3 ou 300 µg/kg sur le comportement des souris a été évalué comme suit :

### Sujets :

Les expériences ont été effectuées sur des souris Swiss mâles, âgées de 6 mois.

### Préparation d'Engrailed-2 injectée:

La protéine EN2 de poulet (Numéro d'accès UniProtKB: Q05917) est produite sous forme recombinante dans des bactéries *E. coli* BL21RP-CodonPlus® (Stratagene) transformées avec le plasmide pGEXSEn2, qui résulte de l'insertion de la séquence codant pour la totalité d'EN2 dans un plasmide pGEX-6P (Amersham-GE Healthcare), en fusion traductionnelle avec la séquence codant pour la glutathion S-transférase (GST).

La protéine exprimée est une protéine de fusion contenant la séquence de la glutathion S-transférase, suivie d'un site de clivage pour la protéase PreScission™ (protéase recombinante résultant de la fusion entre la GST et la protéase 3C du rhinovirus humain ; Amersham-GE Healthcare), et de la séquence complète d'EN2.

Cette protéine de fusion est purifiée à partir d'un lysat des bactéries transformées, par chromatographie d'affinité sur colonne de glutathion (GSTrap, Amershàm-GE Healthcare) ; la protéine EN2 est éluéé de la colonne après clivage à l'aide de la protéase PreScission™.

### Tests effectués

*Labyrinthe en Y :* dans ce test, l'animal est placé dans un labyrinthe en forme d'Y. Chaque visite de l'une des branches du labyrinthe est notée, et le nombre total des visites dans l'une ou l'autre des branches, ainsi que leur succession d'une branche à l'autre sont enregistrés. Le pourcentage d'alternance entre les branches permet d'évaluer la mémoire de travail, le nombre total de visites des branches permet d'évaluer l'activité, et l'évolution de cette activité permet d'évaluer l'habituation à court et à long terme.

*Rotarod :* on mesure le temps pendant lequel l'animal est capable de rester sur un cylindre en rotation, ce qui permet d'évaluer la coordination motrice et l'équilibre.

*Tail-pinch (pincement de la queue) :* ce test permet de mesurer le seuil de douleur.

*Open-field :* L'animal est placé dans une enceinte fortement éclairée, pour une séance de 30 minutes. La distance parcourue et le nombre de redressements permettent d'évaluer l'activité locomotrice et exploratoire ; l'évolution de cette activité entre la première et la seconde moitié du test permet d'évaluer la mémoire de type habituation. Enfin, la détermination du temps passé, et de la distance parcourue, au centre de l'enceinte (qui constitue un environnement anxiogène) permet d'évaluer le niveau d'anxiété.

Le centre de l'open-field est défini comme un carré au centre de l'enceinte représentant 25% de la surface totale. Les variables index du comportement de type anxieux sont le pourcentage de temps passé et le pourcentage de distance parcourue au centre. Le caractère anxiogène du centre est indiqué par un comportement d'évitement vis-à-vis du centre, donc par des valeurs inférieures à 25% pour ces deux variables. Un effet de type anxiolytique d'un traitement se manifeste par une augmentation des pourcentages de temps passé et de distance parcourue au centre.

*Labyrinthe en croix* surélevé : il s'agit d'un dispositif en forme de croix comportant 2 branches ouvertes et 2 branches fermées sur les cotés. L'animal est placé dans le labyrinthe pour une séance de 5 minutes. On mesure le nombre total d'entrées dans les 2 types de branches (activité exploratoire en environnement anxiogène), ainsi que le pourcentage d'entrées dans les branches ouvertes (les plus anxiogènes), et le temps passé dans ces branches. Dans la majorité des cas, la souris passe moins de 50% du temps et réalise moins de 50% des entrées dans les branches ouvertes, ce qui atteste du caractère anxiogène de celles-ci. Un comportement de type anxieux est donc considéré comme d'autant plus prononcé que les pourcentages de temps et d'entrées dans les branches ouvertes sont bas. L'effet de type anxiolytique d'un traitement se manifeste par une augmentation des **pourcentages de temps et d'entrées dans les branches ouvertes.**

### Mode opératoire :

Les injections ont été faites selon le protocole suivant :
Vingt et un animaux ont été répartis au hasard en 4 groupes, qui ont reçu des injections de :
NaCl 0,9% : groupe témoin (Sal) ; n= 10) ;
EN2 3 µg/kg : groupe EN3 (n= 10) ;
EN2 300 µg/kg : groupe EN300 (n= 10).

Les injections sont réalisées par voie intra-veineuse au niveau de la queue.

Le calendrier des injections et des tests est le suivait :

| | | |
|---|---|---|
| Jour 1 : | t= 0 : | Injection 1 |
| | t= 1h | Labyrinthe en Y - Séance 1 |
| Jour 2 : | t= 0 : | Injection 2 |
| | t= 1 h : | Labyrinthe en Y - Séance 2 |
| | t= 1h40 ; | Rotarod - Séance 1 |
| Jour 3 : | t= 0 : | Injection 3 |
| | t= 1h : | Labyrinthe en Y - Séance 3 |
| | t= 1h40 ; | Rotarod - Séance 2 |
| Jour 4 : | t= 0 : | Injection 4 |
| | t= 1h : | Labyrinthe en Y - Séance 4 |
| | t= 1h40 ; | Rotarod - Séance 3 |
| | t= 1h55 ; | Tail-pinch - Séance 1 |
| Jour 7 : | t= 0 : | Injection 5 |
| | t= 1 h : | Open-Field |
| | t= 1h40 ; | Rotarod - Séance 4 |
| | t= 1h55 ; | Tail Pinch - Séance 2 |
| Jour 8 : | t= 0 : | Injection 6 |
| | t= 1h : | Labyrinthe en croix surélevé |

### Résultats :

### Activité locomotrice :

On ne constate pas d'effet notable d'EN2 à 3 et 300 µg/kg. ni pour le labyrinthe, ni pour le rotarod, ni pour le test tail-pinch, ni en open-field. On ne peut donc pas conclure à la présence d'un effet d'En2 sur l'activité locomotrice et exploratoire dans les conditions expérimentales utilisées.

### Mémoire de type habitation :

L'habituation à un environnement se manifeste par une activité exploratoire qui diminue quand cet environnement devient connu. Les variables index d'habituation sont les activités horizontale et verticale, mesurées sur la 2^{éme} moitié d'une séance en open-field, et exprimées en pourcentage de l'activité sur la totalité de la séance. L'habituation est indiquée par des valeurs pour ces deux variables inférieures à 50%.

Le pourcentage de distance au centre et de redressements à la 2^{ème} moitié de la séance est significativement inférieur à 50% chez les trois groupes. En2 ne modifie pas significativement ces deux variables. On ne peut pas conclure à la présence d'un effet d'EN2 sur la mémoire de type habituation dans les conditions expérimentales utilisées.

### Activité anxiolytique :

### Open field

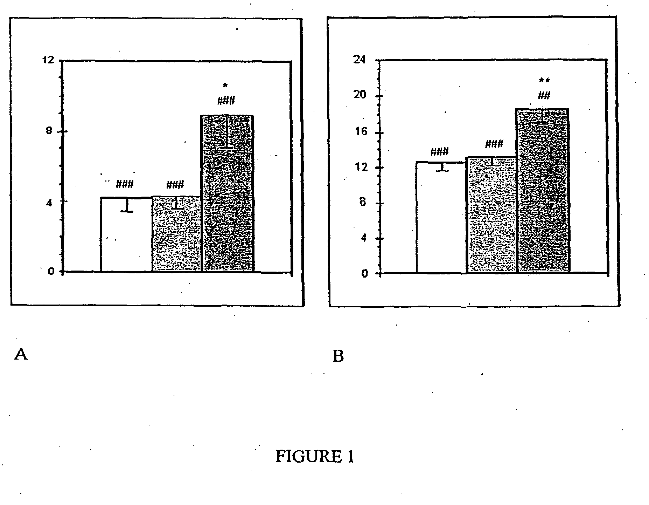

Les résultats sont illustrés par la Figure 1, qui représente le pourcentage de temps passé au centre (A), et le pourcentage de distance parcourue au centre (B), pour les souris témoins (Sal ; □ ), les souris ayant reçu une injection de 3 µg/kg (□), et les souris ayant reçu une injection de 300 µg/kg (□) d' EN2. Différence vs. 25% : ## p<0,01 ; ### p<0,001.

Différence vs. groupe témoin (Sal) : * p<0,05 ; ** p<0,01.

On constate que, pour les 3 groupes d'animaux, les pourcentages de temps passé et de distance parcourue au centre sont inférieurs à 25%, ce qui montre un évitement du centre, indiquant le caractère anxiogène de celui-ci.

A la dose de 3µg/kg, EN2 n'a pas d'effet significatif sur le comportement des animaux ; en revanche, à la dose de 300 µg/kg, EN2 augmente de façon très significative (p<0,01) le pourcentage de temps passé et le pourcentage de distance parcourue au centre, ce qui montre un effet de type anxiolytique.

### Labyrinthe en croix surélevé

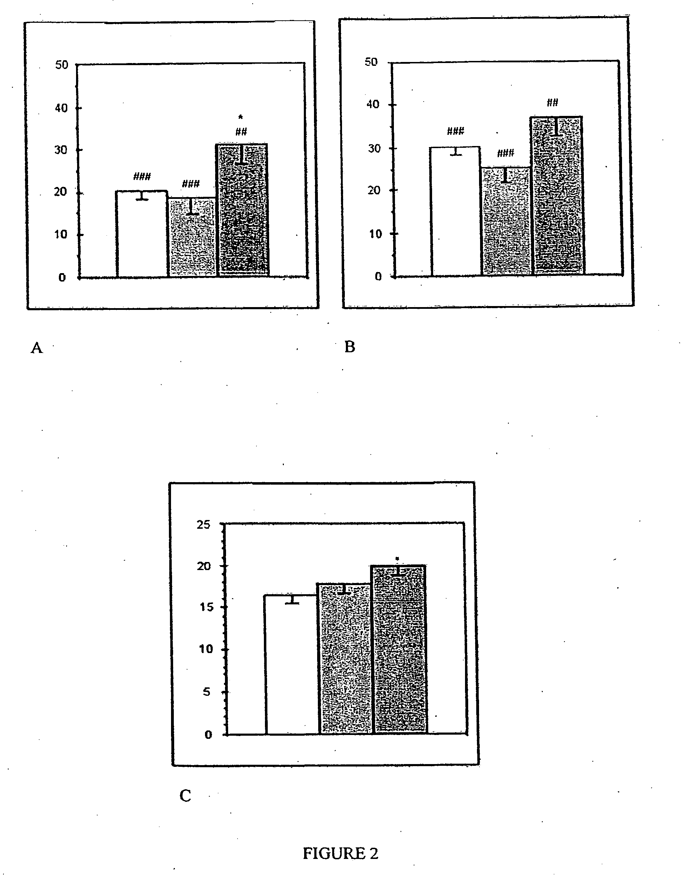

Les résultats sont illustrés par la Figure 2, qui représente le nombre total d'entrées dans les branches ouvertes ou fermées (A), le pourcentage de temps dans les branches ouvertes (B) et le pourcentage d'entrées dans ces branches (C), pour les souris témoins (Sal ; □), les souris ayant reçu une injection de 3 µg/kg ( □ ) d'EN2, et les souris ayant reçu une injection de 300 µg/kg ( □ ) d'EN2. Différence vs. 50% : ## p<0,01 ; ### p<0,001. Différence vs. groupe témoin (Sal) : * p<0,05.

Les trois groupes d'animaux présentent des pourcentages de temps et d'entrées dans les branches ouvertes inférieurs à 50%, ce qui dénote le caractère anxiogène des branches ouvertes.

A la dose de 3 µg/kg, EN2 n'a pas d'effet significatif sur le comportement des animaux.

A la dose de 300 µg/kg, EN2 augmente le nombre d'entrées dans les branches, ouvertes ou fermées, ce qui suggère une augmentation du comportement exploratoire et/ou une diminution de l'anxiété, et augmente également significativement le pourcentage de temps passé dans les branches ouvertes.

Les résultats de ces expériences indiquent qu'un traitement avec la protéine EN2 induit un effet de type anxiolytique.

### EXEMPLE 2 : EFFET D'ENGRAILED-2 SUR LES AMINES CEREBRALES.

Le lendemain des essais décrits dans l'Exemple 1, les souris du groupe témoin et du groupe EN300 ont reçu une 7^{ème} injection, respectivement de NaCl 0,9% et d' EN2 à la dose de 300 µg/kg.

Une heurte après l'injection, les animaux sont sacrifiés par décapitation. Les cerveaux sont rapidement prélevés et placés sur une platine réfrigérée (0-4°C). Les structures cérébrales (cortex, striatum, et hippocampe) sont disséquées, pesées et placées dans un tube Eppendorf à -30°C avant préparation des échantillons de chromatographie.

Les tissus sont homogénéisés avec un potter en teflon dans 500 µl d'une solution d'acide perchlorique 0,1 N contenant 0,05% de métabisulfite de sodium. Les échantillons sont centrifugés (25000 x g, 30 min, 4°C). Les surnageants sont prélevés puis congelés avant le passage en chromatographie.

La dopamine (DA) ainsi qu'un de ses métabolites, l'acide 3,4-dihydroxyphénylacétique (DOPAC), et la sérotonine, sont dosés dans ces surnageants par chromatographie en phase liquide couplée à la spectrométrie de masse (CPL-SM).

Les composés sont séparés par chromatographie de partage en phase inverse, sur une colonne de silice greffée C18 ; l'élution est effectuée avec un mélange Méthanol/Eau : 15/85.

La détection est effectuée à l'aide d'un spectromètre de masse LCQ (ThermoFinnigan). Il s'agit d'une trappe ionique équipée d'une source d'ionisation de type Electrospray fonctionnant en mode d'ionisation positif.

Les résultats sont illustrés dans les Tableaux I, II, et III ci-dessous.

**TABLEAU I**

| STRIATUM | DA (ng/mg tissu) | 5HT (ng/mg) | DOPAC (ng/mg) | DOPAC/DA |
|---|---|---|---|---|
| Contrôle (10) | Moy 15,42 | Moy 1,42 | Moy 6,36 | Moy 0,41 |
| | ESM 1,45 | ESM 0,29 | ESM 0,54 | ESM 0,03 |
| Traitées (10) | Moy 14,08 | Moy 1,14 | Moy 8,33 | Moy 0,62 |
| | ESM 1,70 | ESM 0,19 | ESM 1,15 | ESM 0,08 |
| | ns | ns | p < 0,1 | p < 0,05 |

**TABLEAU II**

| CORTEX | DA (ng/mg tissu) | 5HT (ng/mg) | DOPAC (ng/mg) | DOPAC/DA |
|---|---|---|---|---|
| Contrôle (10) | Mol 1,11 | Moy 2,42 | Moy 5,89 | Moy 5,57 |
| | ESM 0,05 | ESM 0,21 | ESM 0,22 | ESM 0,35 |
| Traitées (10) | Moy 1,25 | Moy 3,10 | Moy 6,17 | Moy 4,97 |
| | ESM 0,08 | ESM 0,26 | ESM 0,26 | ESM 0,49 |
| | ns | p < 0,1 | ns | ns |

**TABLEAU III**

| HIPPOCAMPE | DA (ng/mg tissu) | 5HT (ng/mg) | DOPAC (ng/mg) | DOPAC/DA |
|---|---|---|---|---|
| Contrôle (10) | Moy 0,12 | Moy 0,28 | Moy 0,51 | Moy 3,74 |
| | ESM 0,01 | ESM 0,06 | ESM 0,06 | ESM 0,30 |
| Traitées (10) | Moy 0,12 | Moy 0,28 | Moy 0,43 | Moy 3,93 |
| | ESM 0,02 | ESM 0,06 | ESM 0,09 | ESM 0,24 |
| | ns | ns | ns | ns |

On observe une légère augmentation de la DOPAC et une forte augmentation (50% p<0,05) du rapport DOPAC/DA dans le striatum, ainsi qu'une légère augmentation de la 5HT dans le cortex.

## Revendications

1. Protéine Engrailed pour l'utilisation comme médicament anxiolytique.

2. Protéine Engrailed pour l'utilisation selon la revendication 1, **caractérisée en ce que** ledit médicament est sous forme de préparation injectable.

## Claims

1. Engrailed protein for use as an anxiolytic medicament.

2. Engrailed protein for use as claimed in claim 1, **characterised in that** said medicament is in the form of an injectable preparation.

## Patentansprüche

1. Engrailed-Protein zur Verwendung als angstlösendes Medikament.

2. Engrailed-Protein zur Verwendung gemäß Anspruch 1, **dadurch gekennzeichnet , dass** das Medikament in Form eines injizierbaren Präparates vorliegt.
